# EUROPEAN PATENT APPLICATION

(11) **EP 3 827 790 A1**
(43) Date of publication of application: **02.06.2021**
(21) Application number: 19840086.3
(22) Date of filing: 22.07.2019
(51) Int. Cl.: A61F 2/66

(54) **SOLE FOR PROSTHETIC LEG**

(30) Priority: 24.07.2018 JP 2018138766
(71) Applicant: Bridgestone Corporation, Chuo-Ku Tokyo 104-8340 (JP)
(72) Inventor: ITOI, Dyta, Tokyo 104-8340 (JP); KIWAKI, Yukihiro, Tokyo 104-8340 (JP); ODAIRA, Miho, Tokyo 104-8340 (JP)
(74) Representative: Oxley, Robin John George
(86) International application number: PCT/JP2019/028688
(87) International publication number: WO 2020/022276

(57) **Abstract**

A sole for a prosthetic leg, attached to a ground contact region of the prosthetic leg, includes a bottom surface including: a first inclined surface portion including a first inclined surface inclined such that an amount of recess in a sole thickness direction gradually increases from one to the other sides in a predetermined direction in a planar view; and a second inclined surface portion including a second inclined surface inclined such that the amount of recess in the sole thickness direction gradually increases from the other to the one sides in the predetermined direction in the planar view. The first and second inclined surface portions are mutually connected in a connecting direction crossing the predetermined direction in the planar view, so that a continuous groove in which the first and second inclined surfaces constitute a part of a side wall and which extends in the connecting direction is defined.

## Description

### TECHNICAL FIELD

The present disclosure relates to a sole for a prosthetic leg which is attached to a ground contact region of the prosthetic leg.

### BACKGROUND

Conventionally, a prosthetic leg for athletics having a leaf-spring-like leg portion which extends to a side of a toe via a curved portion and in which a ground contact region extends from the toe to a side of the curved portion in an arc has been well-known. To such a prosthetic leg having the leaf-spring-like leg portion, generally, a sole for a prosthetic leg which abuts a road surface is attached to a bottom surface of the ground contact region.

For example, Patent Literature 1 illustrates a sole attached to a lower surface of a curved leaf-spring-like prosthetic leg to correspond to sporting events such as jogging or running. In other words, Patent Literature 1 discloses a configuration in which a spike is attached at a lower surface of a sole contacting a road surface or a configuration in which a number of outsole portions each having a hexagonal contact patch are provided.

### CITATION LIST

### Patent Literature

PTL 1: Japanese Patent Laid-Open No. 2016-150189

### SUMMARY

### (Technical Problem)

For a prosthetic leg such as an athletic prosthetic leg, anti-slip performance which can inhibit slip and wear resistance performance which improves durability have been required. However, there is still room for improvement.

An object of the present disclosure is to provide a sole for a prosthetic leg which can achieve both anti-slip performance and wear resistance performance.

### (Solution to Problem)

According to a first aspect of the present disclosure, there is provided a sole for a prosthetic leg, the sole being configured to be attached to a ground contact region of the prosthetic leg, the sole including: a bottom surface including: a first inclined surface portion including a first inclined surface inclined such that an amount of recess in a sole thickness direction gradually increases from one side to the other side in a predetermined direction in a planar view; and a second inclined surface portion including a second inclined surface inclined such that the amount of recess in the sole thickness direction gradually increases from the other side to the one side in the predetermined direction in the planar view, wherein the first inclined surface portion and the second inclined surface portion are mutually connected in a connecting direction crossing the predetermined direction in the planar view, so that a continuous groove in which the first inclined surface and the second inclined surface constitute a part of a side wall and which extends in the connecting direction is defined.

### (Advantageous Effect)

According to the present disclosure, a sole for a prosthetic leg which can achieve both anti-slip performance and wear resistance performance can be provided.

### BRIEF DESCRIPTION OF THE DRAWINGS

In the accompanying drawings:
FIG. 1 is a side view of an athletic prosthetic leg to which a sole as one embodiment of the present disclosure is attached;
FIG. 2 is a plan view which illustrates a pattern of a bottom surface of the sole illustrated in FIG. 1;
FIG. 3 is a side view which illustrates a side surface of the sole illustrated in FIG. 1;
FIG. 4 is a cross-sectional view taken along the line I-I of FIG. 2;
FIG. 5A is a drawing for explaining in stages movement of a leg portion and a ground contact state of the bottom surface of the sole in a case where the athletic prosthetic leg illustrated in FIG. 1 is worn and a wearer executes straight running;
FIG. 5B is a drawing for explaining in stages the movement of the leg portion and the ground contact state of the bottom surface of the sole in a case where the athletic prosthetic leg illustrated in FIG. 1 is worn and the wearer executes straight running;
FIG. 5C is a drawing for explaining in stages the movement of the leg portion and the ground contact state of the bottom surface of the sole in a case where the athletic prosthetic leg illustrated in FIG. 1 is worn and the wearer executes straight running;
FIG. 5D is a drawing for explaining in stages the movement of the leg portion and the ground contact state of the bottom surface of the sole in a case where the athletic prosthetic leg illustrated in FIG. 1 is worn and the wearer executes straight running;
FIG. 6 is a drawing for explaining each region of the bottom surface of the sole illustrated in FIG. 1;
FIG. 7 is a drawing which illustrates a variation of the pattern of the bottom surface illustrated in FIG. 2; and
FIG. 8 is a drawing which illustrates a sole as another embodiment of the present disclosure.

### DETAILED DESCRIPTION

Hereinafter, with reference to the drawings, an embodiment of a sole for a prosthetic leg according to the present disclosure (hereinafter, it is also referred to as a sole) will be explained with illustration. In each drawing, common members and portions are applied the same reference signs.

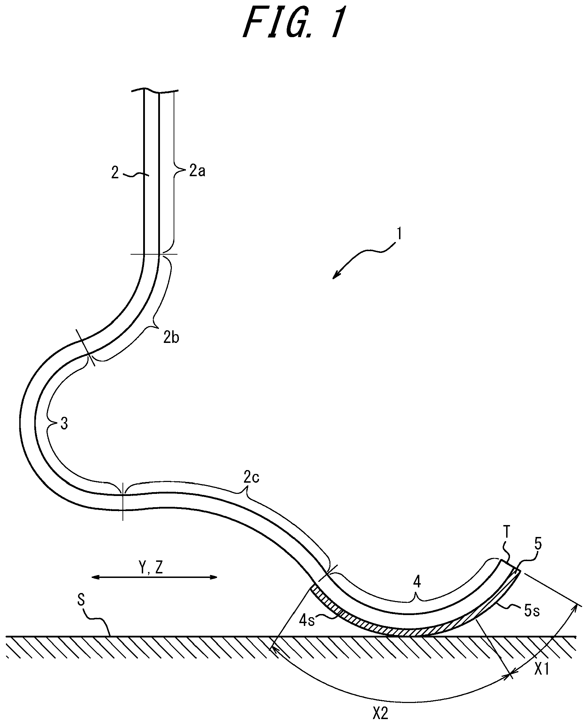

FIG. 1 is a side view which illustrates an athletic prosthetic leg 1 as one example of a prosthetic leg to which a sole 5 for a prosthetic leg according to the embodiment of the present disclosure is attached. The athletic prosthetic leg 1 has a leaf-spring-like leg portion 2, and the sole 5 is attached to a ground contact region at its tip side. Additionally, although not illustrated, a base end portion of the leg portion 2 (an upper end portion in FIG. 1) is connected to a socket via an adapter. A wearer can wear the athletic prosthetic leg 1 by housing a stump of a wearer's leg in the socket. The adapter and the socket which correspond to the position of the stump of the leg, such as an above-knee prosthesis and a below-knee prosthesis, are used. FIG. 1 illustrates the leg portion 2 and the sole 5 in a standing state of the wearer who wears the athletic prosthetic leg 1.

Hereinafter, in this embodiment, in the athletic prosthetic leg 1, a side where the leg portion 2 is connected to the adapter (an upper side in FIG. 1) is defined as a connection side, and a side where the leg portion 2 contacts a road surface S (a lower side in FIG. 1) is defined as a ground contact side. Also, a toe T of the athletic prosthetic leg 1 refers to a point at the forefront as a termination of the leg portion 2 extending from the connection side. Further, a direction extending from the toe T in parallel with the road surface S is defined as a leg portion front-rear direction Y (the same direction as a sole front-rear direction Z at the time of standing). Further, a widthwise direction of the leg portion 2 is defined as a width direction W (also referred to as a sole width direction W).

In this embodiment, the leg portion 2 of the athletic prosthetic leg 1 has a plate-like extending shape to the side of the toe T via at least one curved portion, in the illustrated example, one curved portion 3. In FIG. 1, the leg portion 2 is constituted by, in the order from the connection side to the ground contact side, a straight portion 2a, a curved portion 2b which is convex to the side of the toe T, the curved portion 3 which is convex to a rear side in the leg portion front-rear direction Y, a curved portion 2c which is concave to the ground contact side and a ground contact portion 4 which is convex to the ground contact side to extend to the side of the toe T in an arc.

Additionally, although the material of the leg portion 2 is not limited, from a viewpoint of strength and weight saving, fiber reinforced plastic etc. is preferably used.

The ground contact portion 4 includes a ground contact region 4s extending from the toe T to the side of the curved portion 3 in an arc at the ground contact side, and the sole 5 is attached to the ground contact region 4s. The ground contact region 4s refers to the entire region abutting the road surface S when the wearer who wears the athletic prosthetic leg 1 executes straight running movement in a state that the sole 5 is not attached to the athletic prosthetic leg 1.

The sole 5 can be deformed along the ground contact region 4s of the athletic prosthetic leg 1, and is attached in a deformed state along an extending shape of the ground contact region 4s. Also, as illustrated in FIG. 1, a bottom surface 5s which is the ground contact side of the sole 5 has a shape in which an arc X1 and an arc X2 are continued from the toe T side to the curved portion 3 side in a side view. While the arc X1 and the arc X2 have a different radius of curvature to each other in this embodiment, they may have the same radius of curvature.

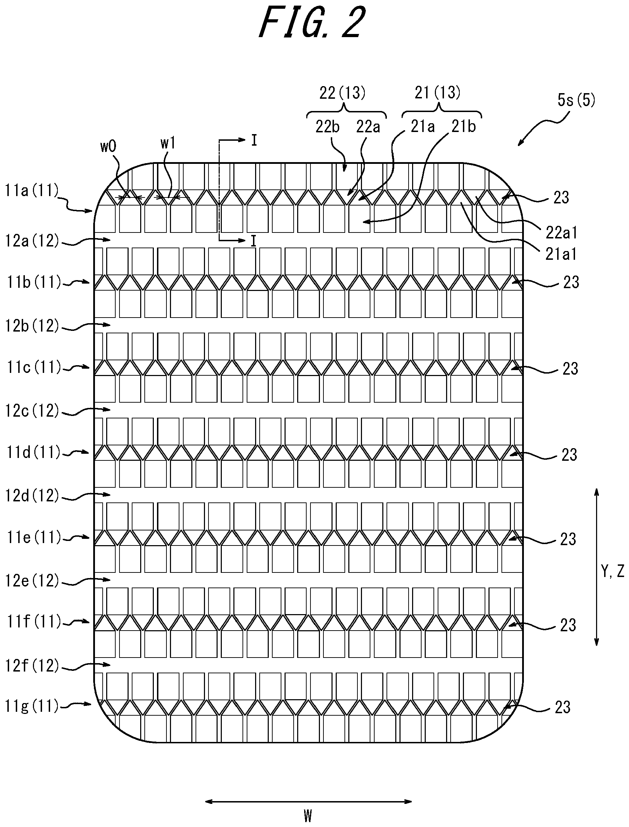

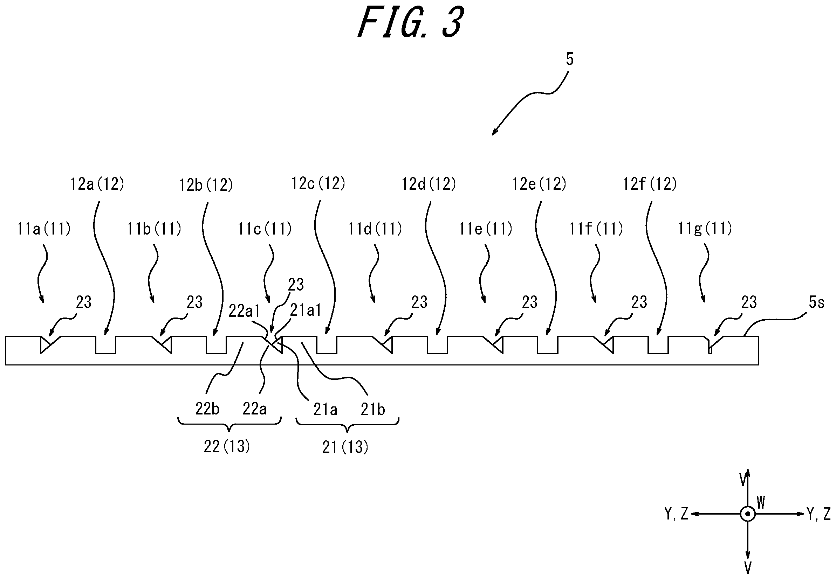

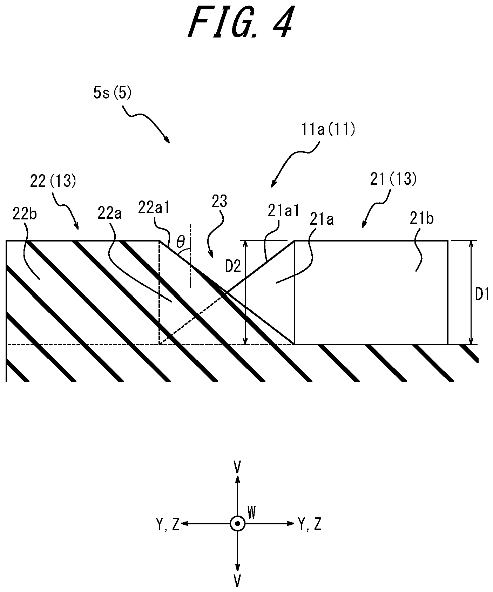

FIG. 2 is a plan view which illustrates the bottom surface 5s of the sole 5. FIG. 3 is a side view of the sole 5. FIG. 4 is a cross-sectional view taken along the line I-I of FIG. 2. As illustrated in FIGS. 2 to 4, a pattern constituted by recesses and protrusions is formed on the bottom surface 5s of the sole 5. Here, for convenience of explanation, a direction which is a side of the toe T of the sole 5 (see FIG. 1) in a state that the sole 5 is attached to the athletic prosthetic leg 1 as a prosthetic leg is described as "a front side" in a sole front-rear direction Z, and a direction which is a heel side of the sole 5 is described as "a rear side" in the sole front-rear direction Z. Hereinafter, the pattern formed on the bottom surface 5s will be explained.

As illustrated in FIGS. 2 and 3, a plurality of belt-like land portion rows 11 (seven in this embodiment) each being constituted by a plurality of land portions 13 are provided on the bottom surface 5s of this embodiment. More specifically, the plurality of land portion rows 11 of this embodiment are constituted by seven land portion rows 11a to 11g extending in the sole width direction W at different positions in the sole front-rear direction Z. More specifically, the seven land portion rows 11a to 11g defined by six width direction grooves 12a to 12f extending to a front end edge and a rear end edge in the sole front-rear direction Z and in the sole width direction W are provided on the bottom surface 5s of this embodiment. Hereinafter, when the seven land portion rows 11a to 11g are not particularly differentiated, they are simply described as "a land portion row 11". Also, when the six width direction grooves 12a to 12f are not particularly differentiated, they are simply described as "a width direction groove 12".

As described above, the land portion row 11 of the bottom surface 5s is constituted by the plurality of land portions 13. Each of the plurality of land portions 13 includes a first land portion 21 having a first inclined surface portion 21a and a second land portion 22 having a second inclined surface portion 22a.

As illustrated in FIG. 4, the first inclined surface portion 21a of the first land portion 21 includes a first inclined surface 21a1 which is inclined such that an amount of recess in a sole thickness direction V gradually increases from one side to the other side in a predetermined direction in a planar view (see FIG. 2). More specifically, the first inclined surface 21a1 of the first inclined surface portion 21a of the first land portion 21 of this embodiment is inclined such that the amount of recess in the sole thickness direction V gradually increases from a rear side toward a front side in the sole front-rear direction Z in the planar view (see FIG. 2).

As illustrated in FIG. 4, the second inclined surface portion 22a of the second land portion 22 includes a second inclined surface 22a1 which is inclined such that the amount of recess in the sole thickness direction V gradually increases from one side to the other side in the above-described predetermined direction in the planar view (see FIG. 2). More specifically, the second inclined surface 22a1 of the second inclined surface portion 22a of the second land portion 22 of this embodiment is inclined such that the amount of recess in the sole thickness direction V gradually increases from the front side toward a rear side in the sole front-rear direction Z in the planar view (see FIG. 2).

More specifically, the first inclined surface portion 21a and the second inclined surface portion 22a of this embodiment are inclined surface portions which have a symmetrical shape to a virtual line extending in the sole thickness direction V and includes inclined surfaces which are inclined oppositely to each other. Such first inclined surface portion 21a and second inclined surface portion 22a are provided to the bottom surface 5s.

Here, the first inclined surface portion 21a and the second inclined surface portion 22a are mutually connected in a connecting direction crossing the above-described predetermined direction in the planar view (see FIG. 2). The above-described predetermined direction of this embodiment is the sole front-rear direction Z. Also, the connecting direction crossing the above-described predetermined direction of this embodiment is the sole width direction W. Consequently, the first inclined surface portion 21a and the second inclined surface portion 22a of this embodiment are mutually connected in the sole width direction W in the planar view (see FIG. 2). Additionally, "connecting" means a state that a plurality of members and portions are continuously connected, and here, means a state that the first inclined surface portion 21a the second inclined surface portion 22a are continuously connected such that at least they mutually abut in a state of contacting the road surface S (see FIG. 1) at the time of straight running.

As illustrated in FIGS. 2 to 4, on the bottom surface 5s, a continuous groove 23 extending in the connecting direction is defined, in which the first inclined surface portion 21a and the second inclined surface portion 22a are mutually connected in the sole width direction W as the connecting direction, so that the first inclined surface 21a1 and the second inclined surface 22a1 constitute a part of a side wall.

In other words, the first inclined surface portion 21a and the second inclined surface portion 22a are arranged such that they mutually overlap in the sole width direction W as the connecting direction, whereby the continuous groove 23 extending in the sole width direction W as the connecting direction can be formed.

By arranging the first inclined surface portion 21a and the second inclined surface portion 22a as described above, the above-described continuous groove 23 can be formed. Accordingly, compared with a configuration in which a simple recess and protrusion pattern constituted by a plurality of long grooves and belt-like land portions formed between the plurality of long groves is arranged on a bottom surface, drainage performance can be improved. This improves anti-slip performance of the bottom surface 5s. Also, in the bottom surface 5s, since the first inclined surface 21a1 and the second inclined surface 22a1 are provided, compared with a configuration in which an orthogonal plane which is orthogonal to the bottom surface 5s is provided instead of the first inclined surface 21a1 and the second inclined surface 22a1, collapsing of the first inclined surface portion 21a and the second inclined surface portion 22a is difficult to occur also in a direction in which the amount of recess in the sole thickness direction V gradually increases (in this embodiment, the front side or the rear side in the sole front-rear direction Z), and rigidity in the direction in which the amount of recess in the sole thickness direction V gradually increases (in this embodiment, the front side or the rear side in the sole front-rear direction Z) can be improved. Consequently, wear resistance performance of the bottom surface 5s in the direction in which the amount of recess in the sole thickness direction V gradually increases (in this embodiment, the front side or the rear side of the sole front-rear direction Z) can be improved. Further, the inclined surface portions (the first inclined surface portion 21a and the second inclined surface portion 22a) which are adjacent in the connecting direction (in this embodiment, the sole width direction W) are mutually supported at the time of ground contact. Due to this, collapsing of the inclined surface portion in the connecting direction can also be inhibited, and rigidity in the connecting direction (in this embodiment, the sole width direction W) is difficult to be lowered. As described above, by providing the first inclined surface portion 21a and the second inclined surface portion 22a to the bottom surface 5s, both anti-slip performance and wear resistance performance of the bottom surface 5s can be achieved.

In the bottom surface 5s of this embodiment, the plurality of first land portions 21 each including the first inclined surface portion 21a and the plurality of second land portions 22 each including the second inclined surface portion 22a are connected in the sole width direction W, whereby the seven land portion rows 11a to 11g are formed. However, the number of the land portion rows 11 is not particularly limited. The number of the land portion rows 11 may be six or less, or may be eight or more. The number of width direction grooves 12 can be appropriately changed in accordance with the number of the land portion rows 11. Also, while the depth of the width direction groove 12 is not particularly limited, a depth D1 of the width direction groove 12 is a depth D2 of the continuous groove 23 or more. As illustrated in FIG. 4, the depth D1 of the width direction groove 12 of this embodiment is equal to the depth D2 of the continuous groove 23.

In the bottom surface 5s of this embodiment, the plurality of first land portions 21 each including the first inclined surface portion 21a and the plurality of second land portions 22 each including the second inclined surface portion 22a are connected in the sole width direction W, whereby the land portion row 11 is formed. However, this is not limited to a configuration of forming the land portion row 11 as long as the first inclined surface portion 21a and the second inclined surface portion 22a are connected such that the continuous groove 23 is defined. Also, in this embodiment, the first inclined surface portion 21a and the second inclined surface portion 22a are connected in the sole width direction W such that the continuous groove 23 extends in the sole width direction W. However, not limited to this configuration, for example, the first inclined surface portion 21a and the second inclined surface portion 22a may be connected in the sole front-rear direction Z such that the continuous groove 23 extends in the sole front-rear direction Z. Additionally, as in this embodiment, a direction in which the amount of recess in the sole thickness direction V of the first inclined surface 21a1 and the second inclined surface 22a1 gradually increases is preferably the sole front-rear direction Z. In other words, as in this embodiment, the first inclined surface portion 21a and the second inclined surface portion 22a are preferably connected in the sole width direction W such that the continuous groove 23 extends in the sole width direction W. By doing this, rigidity in the sole front-rear direction Z can be further improved by the first inclined surface portion 21a and the second inclined surface portion 22a. As a result, wear resistance performance in the sole front-rear direction Z can be further improved. This achieves the bottom surface 5s which is difficult to be worn even with the straight running movement.

The continuous groove 23 of this embodiment extends in the sole width direction W. More specifically, the continuous groove 23 of this embodiment extends over the entire region in the sole width direction W of the bottom surface 5s, and continues to an outer edge in the sole width direction W of the bottom surface 5s. With this configuration, drainage performance of the bottom surface 5s can be further improved.

Also, in the bottom surface 5s of this embodiment, the plurality of first inclined surface portions 21a and the plurality of second inclined portions 22a are connected. However, the number of the first inclined surface portions 21a and the number of the second inclined surface portions 22a are not particularly limited. The plurality of land portions 13 having any form may be applied as long as they include at least one first inclined surface portion 21a and at least one second inclined surface portion 22a to define the continuous groove 23.

Further, in the bottom surface 5s of this embodiment, the first inclined surface portion 21a and the second inclined surface portion 22a are alternately connected. However, this is not limited to the configuration of alternate connection. The first inclined surface portion 21a and the second inclined surface portion 22a of this embodiment have outer triangular shapes directed oppositely to each other in the sole front-rear direction Z in the planar view (see FIG. 2). Consequently, in this embodiment, the alternate connection is required to connect the first inclined surface portion 21a and the second inclined surface portion 22a in the sole width direction W. However, the outer shape of the first inclined surface portion 21a and the second inclined surface portion 22a in the planar view (see FIG. 2) is not limited to a triangular shape. Accordingly, depending on the outer shape of the first inclined surface portion 21a and the second inclined surface portion 22a in the planar view (see FIG. 2), configurations other than a configuration that they are alternately connected may be applied. For example, a configuration that two connected first inclined surface portions 21a and two connected second inclined surface portions 22a are alternately connected may be applied.

A width w0 in the sole width direction W of the first inclined surface portion 21a of this embodiment gradually decreases toward the front side in the sole front-rear direction Z in the planar view (see FIG. 2). More specifically, as described above, the first inclined surface portion 21a of this embodiment has a triangular shape having an apex at the front side in the sole front-rear direction Z in the planar view (see FIG. 2). Also, a width w1 in the sole width direction W of the second inclined surface portion 22a of this embodiment gradually decreases toward the rear side in the sole front-rear direction Z in the planar view (see FIG. 2). More specifically, as described before, the second inclined surface portion 22a of this embodiment has a triangular shape having an apex at the rear side in the sole front-rear direction Z in the planar view (see FIG. 2). In this way, in this embodiment, the first inclined surface portion 21a and the second inclined surface portion 22a have a configuration that the width w0 and the width w1 gradually decrease toward the direction in which the amount of recess in the sole thickness direction V gradually increases (in this embodiment, the front side or the rear side in the sole front-rear direction Z) crossing the connecting direction (in this embodiment, the sole width direction W) in the planar view (see FIG. 2). Also, in the first inclined surface portion 21a and the second inclined surface portion 22a of this embodiment, the directions in which the amount of recess in the sole thickness direction V gradually increases are mutually opposite. Consequently, the first inclined surface portion 21a and the second inclined surface portion 22a are easily connected not only in the sole width direction W as the connecting direction but also in the sole front-rear direction Z as a direction crossing the connecting direction. In other words, the first inclined surface portion 21a and the second inclined surface portion 22a can be mutually supported also in the sole front-rear direction Z at the time of ground contact, which further improves rigidity in the sole front-rear direction Z. As a result, wear resistance performance of the bottom surface 5s can be further improved. Additionally, the outer shape of the first inclined surface portion 21a and the second inclined surface portion 22a in the planar view (see FIG. 2) is not limited to the triangular shape of this embodiment, and the same effect as the above can be obtained even with a trapezoidal shape.

The first land portion 21 of this embodiment includes a first body portion 21b which is substantially rectangular in the planar view (see FIG. 2) and has a top surface which contacts the road surface S (see FIG. 1) and the first inclined surface portion 21a which includes the above-described first inclined surface 21a1 which continues from this first body portion 21b to the front side in the sole front-rear direction Z. The shape of the first body portion 21b in the planar view (see FIG. 2) is not limited to substantially rectangular of this embodiment, and other outer shapes such as circular may be applied. Also, while the first body portion 21b has a configuration having a flat top surface, a curved top surface including a recess or a protrusion may be applied.

The second land portion 22 of this embodiment includes a second body portion 22b which is substantially rectangular in the planar view (see FIG. 2) and has a top surface which contacts the road surface S (see FIG. 1) and the second inclined surface portion 22a which includes the above-described second inclined surface 22a1 which continues from this second body portion 22b to the front side in the sole front-rear direction Z. The shape of the second body portion 22b in the planar view (see FIG. 2) is not limited to substantially rectangular of this embodiment, and other shapes such as circular may be applied. Also, while the second body portion 22b has a configuration having a flat top surface, a curved top surface including a recess or a protrusion may be applied.

In this embodiment, the plurality of first land portions 21 are connected in the sole width direction W due to connection of the first body portions 21b in the sole width direction W. However, the configuration of the first land portion 21 is not limited to this configuration. For example, the first body portions 21b which are adjacent in the sole width direction W as illustrated in FIG. 2 may be integrated at a partial or the entire region in the sole width direction W to constitute one or more first land portions 21.
In other words, in the planar view (see FIG. 2), one first land portion 21 including one first body portion 21b extending over the entire region in the sole width direction W and a plurality of first inclined surface portions 21a protruding from different positions in the sole width direction W of the first body portion 21b to the front side in the sole front-rear direction Z may be applied.

Also, in this embodiment, the plurality of second land portions 22 are connected in the sole width direction W due to connection of the second body portions 22b in the sole width direction W. However, the configuration of the second land portion 22 is not limited to this configuration. For example, the second body portions 22b which are adjacent in the sole width direction W as illustrated in FIG. 2 may be integrated at a partial or the entire region in the sole width direction W to constitute one or more second land portions 22. In other words, in the planar view (see FIG. 2), one second land portion 22 including one second body portion 22b extending over the entire region in the sole width direction W and a plurality of second inclined surface portions 22a protruding from different positions in the sole width direction W of the second body portion 22b to the rear side in the sole front-rear direction Z may be applied.

As illustrated in FIG. 4, an inclination angle θ of the first inclined surface 21a1 and the second inclined surface 22a1 to the sole thickness direction V is in a range of preferably 40° to 80°, more preferably 45° to 60°, and most preferably 50° to 55°. By making a range of the inclination angle θ 40° or more, wear resistance performance of the first inclined surface portion 21a and the second inclined surface portion 22a can be improved.
By making the range of the inclination angle θ 80° or less, a depth of the continuous groove 23 defined by the first inclined surface portion 21a and the second inclined surface portion 22a can be ensured, which improves drainage performance.

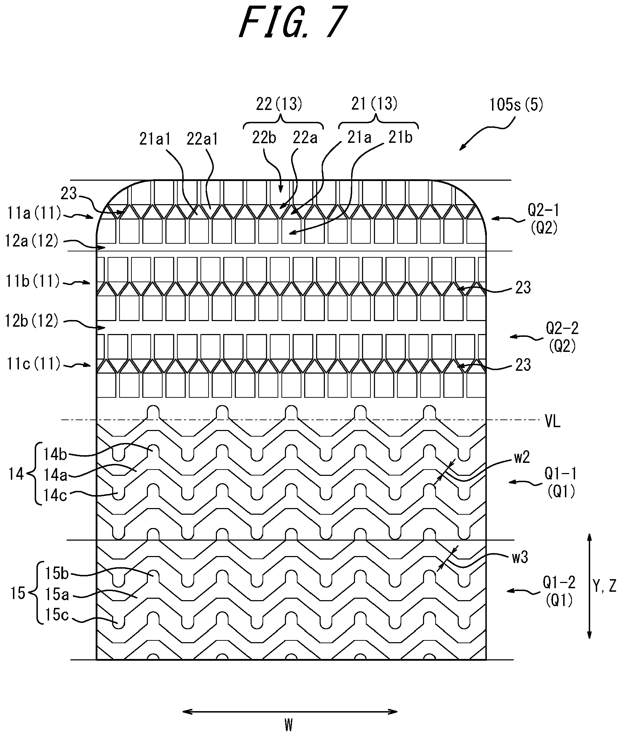

In the bottom surface 5s of this embodiment, as the recess and protrusion pattern, only the land portion row 11 constituted by the plurality of land portions 13 including the first land portion 21 and the second land portion 22 are formed. However, the bottom surface including another recess and protrusion pattern may be applied. The bottom surface including another recess and protrusion pattern will be explained later (see FIG. 7).

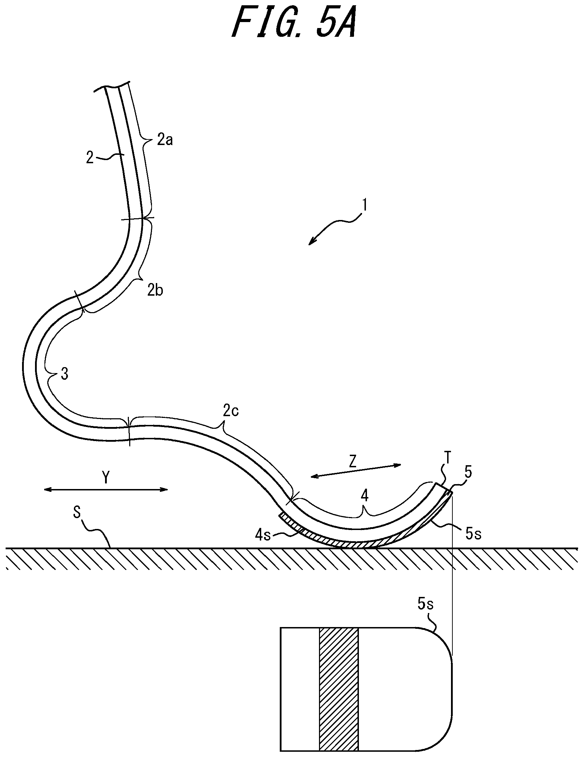

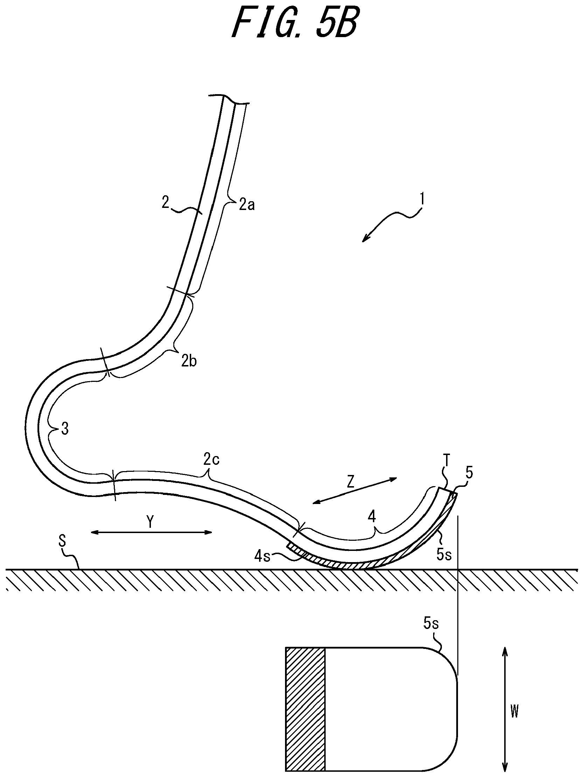

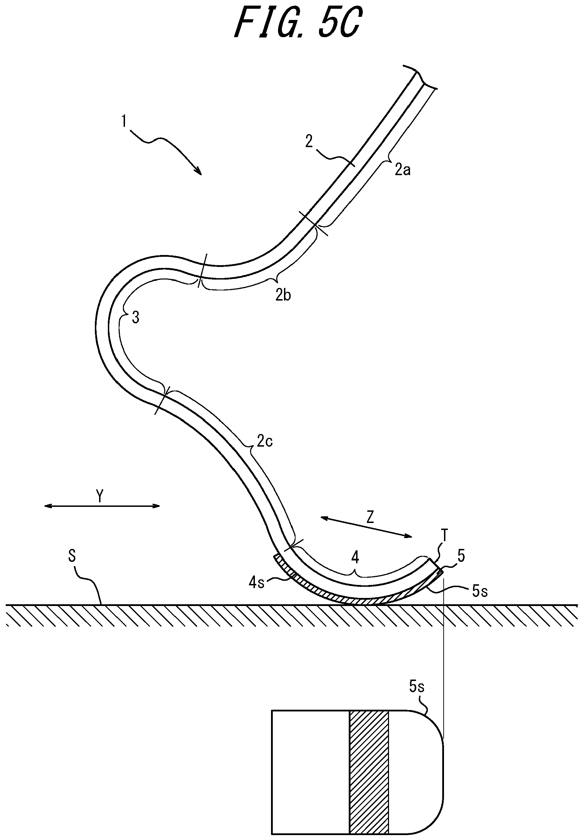

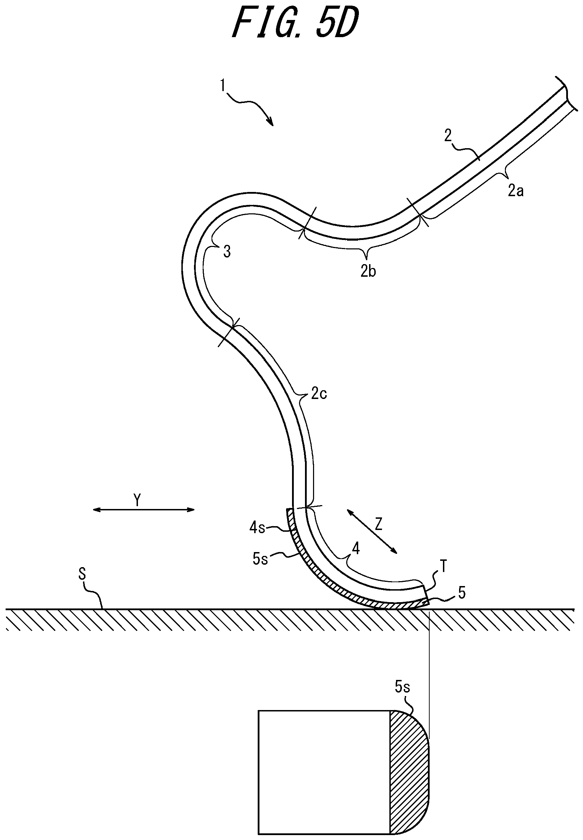

Hereinafter, an example of change of a ground contact state of the bottom surface 5s in the straight running movement of the wearer who wears the athletic prosthetic leg 1 illustrated in FIG. 1 will be explained using FIGS. 5A, 5B, 5C and 5D.

FIGS. 5A, 5B, 5C and 5D are drawings for explaining in stages movement of the leg portion 2 and the ground contact state of the bottom surface 5s of the sole 5 when the wearer who wears the athletic prosthetic leg 1 having the above configuration executes straight running. In each drawing, an upper portion is a side view of the athletic prosthetic leg 1 in each stage in the straight running movement, and a lower portion illustrates a transition of the ground contact region of the bottom surface 5s of the sole 5 in each stage in the straight running movement.

In other words, FIG. 5A illustrates a state that the wearer lowers the raised athletic prosthetic leg 1 to the road surface S and the entire weight is loaded on the athletic prosthetic leg 1 in the straight running movement. In this state, as illustrated in the lower portion of FIG. 5A, a predetermined region in the sole front-rear direction Z of the bottom surface 5s contacts the ground. This predetermined region can be changed by the wearer who wears the athletic prosthetic leg 1. However, when the athletic prosthetic leg 1 illustrated in FIG. 1 is used, the predetermined region at the time of a step movement is a region not including a front end to the side of the toe T in the sole front-rear direction Z as well as not including a rear end at the heel side in the sole front-rear direction Z.

FIG. 5B illustrates a state that the wearer steps forward, from the state of FIG. 5A, while the wearer remains to load the entire weight on the athletic prosthetic leg 1. In a case of running of a healthy person, generally, the ground contact region at the time of straight running movement is sequentially moved from the heel side of a shoe sole which firstly contacts the ground to a toe side, while in the athletic prosthetic leg 1 illustrated in FIG. 1, the ground contact region is moved to the heel side (the side of the curved portion 3) from the region which firstly contacts the ground (see FIG. 5A).

FIG. 5C illustrates a state that the wearer starts a kick-out movement of the athletic prosthetic leg 1 by shaking an opposite leg from the leg wearing the athletic prosthetic leg 1 forward. Entering into this kick-out movement, the ground contact region of the athletic prosthetic leg 1 is moved to a front side region at the side of the toe T in the sole front-rear direction Z of the bottom surface 5s than the predetermined region of the bottom surface 5s which firstly contacts the ground at the time of the step movement (see FIG. 5A).

FIG. 5D illustrates a state of a final stage of kicking out by the athletic prosthetic leg 1 just before the bottom surface 5s of the sole 5 is separated from the road surface S. To kick out from the toe T of the bottom surface 5s, ground contact is executed further at the side of the toe T than in FIG. 5C.

In this way, the athletic prosthetic leg 1 illustrates a unique ground contact form caused by the shape of the leaf-spring-like leg portion 2 in the straight running movement.

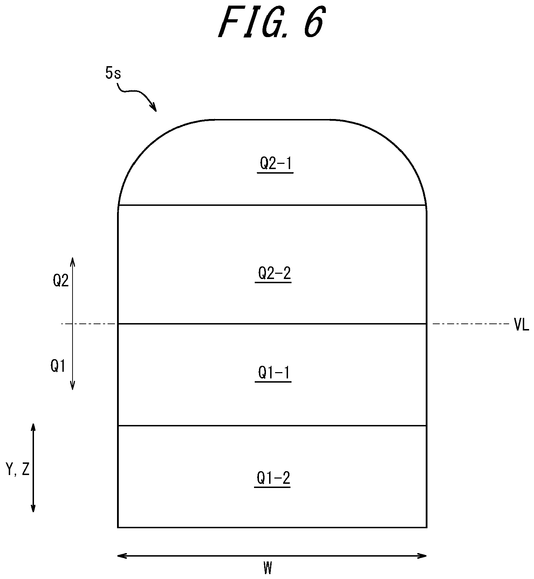

Based on the ground contact form illustrated in FIGS. 5A, 5B, 5C and 5D, as illustrated in FIG. 6, firstly, the bottom surface 5s is divided into a rear side region Q1 and a front side region Q2. When a virtual line VL extending in parallel with the sole width direction W is defined at a center position in the sole front-rear direction Z, the rear side region Q1 and the front side region Q2 are defined using the virtual line VL as a border. More specifically, the rear side region Q1 means a region at the rear side in the sole front-rear direction Z than the virtual line VL. The front side region Q2 means a region at the front side in the sole front-rear direction Z than the virtual line VL. Additionally, FIG. 6 illustrates the bottom surface 5s in a flat state not conforming to the ground contact region 4s of the athletic prosthetic leg 1.

As illustrated in FIGS. 5A and 5B, the rear side region Q1 easily becomes a region which contacts the ground from the start of the step movement by the wearer to before the start of the kick-out movement. Consequently, it is vital that the rear side region Q1 fully grips the road surface S such that the entire body is balanced even when the entire weight of the wearer is loaded on the athletic prosthetic leg 1. Thus, to prevent slip due to a water film interposed between the bottom surface 5s and the road surface S, drainage performance of the rear side region Q1 needs to be higher than a portion other than the rear side region Q1, that is, the front side region Q2. In other words, since the rear side region Q1 has a higher drainage performance compared with that in the portion other than the rear side region Q1, the sole 5 of the athletic prosthetic leg 1 prevents slip due to the water film and achieves high anti-slip performance.

On the other hand, as illustrated in FIGS. 5C and 5D, the front side region Q2 easily becomes a region which contacts the ground after the wearer shakes the opposite leg from the leg wearing the athletic prosthetic leg 1 forward to start the kick-out movement of the athletic prosthetic leg 1. Consequently, the front side region Q2 sequentially contacts the ground toward the toe T, and the wearer presses the road surface S by the bottom surface 5s to slidingly contact the ground, so that the front side region Q2 is a region which easily develops abrasion in particular. Thus, wear resistance performance of the front side region Q2 needs to be higher than that of the rear side region Q1. In other words, with the front side region Q2 having a higher wear resistance performance than the rear side region Q1, early abrasion of the front side region Q2 is avoided, and as a result, the entire surface of the sole 5 of the athletic prosthetic leg 1 is gently worn and a long service life of the sole 5 can be achieved.

From the above, the plurality of land portions 13 each including the above-described first inclined surface portion 21a and the second inclined surface portion 22a may be applied to any of the rear side region Q1 and the front side region Q2 of the bottom surface 5s. Application of the above-described first inclined surface portion 21a and the second inclined surface portion 22a to the rear side region Q1 can improve drainage performance of the rear side region Q1 due to the continuous groove 23. Also, application of the above-described first inclined surface portion 21a and the second inclined surface portion 22a to the front side region Q2 can improve wear resistance performance of the front side region Q2. Additionally, the plurality of land portions 13 each including the above-described first inclined surface portion 21a and the second inclined surface portion 22a are especially excellent in wear resistance performance compared with drainage performance. Consequently, the plurality of land portions 13 each including the above-described first inclined surface portion 21a and the second inclined surface portion 22a are preferably provided at least on the front side region Q2 at the side of the toe T (see FIG. 1) with the border of the virtual line VL. By doing this, a certain degree of drainage performance can be applied also to the front side region Q2 by the continuous groove 23 while wear resistance performance of the front side region Q2 is sufficiently high.

Also, it is preferable that each of the rear side region Q1 and the front side region Q2 is further divided as illustrated in FIG. 6 based on the ground contact form illustrated in FIGS. 5A to 5D such that each portion has property corresponding to the ground contact form.

In other words, of the front side region Q2 illustrated in FIG. 6, a portion Q2-1 corresponds to the arc X1 which continues from the toe T with a constant radius of curvature in FIG. 1. The portion Q2-1 finally contacts the ground when the wearer who wears the athletic prosthetic leg 1 executes the kick-out movement, so that severer abrasion has been inclined to occur. Thus, the portion Q2-1 needs to have an especially high wear resistance performance. In other words, in the front side region Q2, the portion Q2-1 has a higher wear resistance performance than a remaining portion Q2-2, so that the sole 5 is protected from severe abrasion and the long service life of the leg portion 2 itself can be achieved.

As described above, the plurality of land portions 13 each including the first inclined surface portion 21a and the second inclined surface portion 22a are especially excellent in wear resistance performance compared with drainage performance. Consequently, the land portion row 11 which includes the first land portion 21 having the above-described first land portion 21 and the second land portion 22 having the above-described second inclined surface portion 22a are preferably provided at least on a region including the front end in the sole front-rear direction Z of the bottom surface 5s. In other words, the land portion row 11 is preferably provided at least on the portion Q2-1 of the front side region Q2. By doing this, wear resistance performance of the portion Q2-1 can be improved. As a result, the sole 5 is protected from severe abrasion and the long service life of the leg portion 2 itself can be achieved.

Next, in the above-described rear side region Q1, a portion Q1-1 at the side of the toe T in the sole front-rear direction Z is a region which firstly contacts the ground at the time of straight running movement, so that prevention of slip is especially necessary such that the wearer achieves a balance of his body. Thus, the portion Q1-1 preferably has a further higher drainage performance than a remaining portion Q1-2 in the rear side region Q1 such that slip is more surely prevented and a further stable running is achieved.

Also, the portion Q1-2 is a portion at the rear side in the sole front-rear direction Z than the portion Q1-1. In FIG. 6, the portion Q1-2 is a region at the rear side using a center position in the sole front-rear direction Z of the rear side region Q1 as a border. In the rear side region Q1, as illustrated in FIG. 5B, the ground contact region is changed to the rear side in the front-rear direction Z than the portion Q1-1 which firstly contacts the ground, that is, the portion Q1-2 at the opposite side from a direction that the wearer advances. At the time of ground contact of the portion Q1-2, movement of an upper body in which the wearer tries to move forward and a direction in which the ground contact region is moved are temporarily opposite, so that a high propulsive force is needed for the kick-out movement at the latter half of the ground contact form. Consequently, firstly, it is vital that the portion Q1-2 has a higher rigidity than the portion Q1-1. Since the portion Q1-2 has a higher rigidity than the portion Q1-1, the step movement is smoothly continued to the kick-out movement, and a high propulsive force can be achieved.

Especially, in a case where the bottom surface 5s includes a pattern constituted by a plurality of recesses and protrusions, the portion Q1-2 preferably has a larger edge component in the width direction W of the leg portion 2 than the portion Q1-1. Also, a negative ratio of the portion Q1-2 is preferably smaller than that of the portion Q1-1. Here, the negative ratio refers to a percentage in an area of a recessed portion to the road surface S in the planar view in a total area of the bottom surface 5s in the planar view. With this configuration, a high propulsive force can be exerted in running.

Also, to exert the propulsive force effectively, the portion Q1-2 preferably has a larger edge component in the sole width direction W of the leg portion 2 than the front side region Q2. Further, a negative ratio of the portion Q1-2 is preferably larger than that of the front side region Q2. With this configuration, the portion Q1-2 can exert a high propulsive force when the wearer executes the kick-out movement.

From the above, when the land portion row 11 which includes the first land portion 21 having the above-described first inclined surface portion 21a and the second land portion 22 having the above-described second inclined portion 22a is applied to the rear side region Q1, the land portion row 11 is preferably provided at least on the portion Q1-2 of the rear side region Q1. By doing this, rigidity of the portion Q1-2 is improved, and propulsive performance can be improved.

Next, based on the division of a functional region (the portions Q1-1, Q1-2, Q2-1, Q2-2) of the bottom surface 5s illustrated in FIG. 6, a bottom surface 105s having a recess and protrusion pattern which is different from that of the bottom surface 5s illustrated in FIGS. 2 to 4 will be explained with reference to FIG. 7.

In the bottom surface 105s illustrated in FIG. 7, the land portion row 11 constituted by the plurality of land portions 13 each including the first inclined surface portion 21a and the second inclined surface portion 22a is provided only at the front side region Q2 in the sole front-rear direction Z. More specifically, in FIG. 7, three land portion rows 11a to 11c extending in the sole width direction W are provided at the front side region Q2.
Between the three land portion rows 11a to 11c, two width direction grooves 12a, 12b extending in the sole width direction W are arranged. In this way, the land portion row 11 constituted by the plurality of land portions 13 each including the first inclined surface portion 21a and the second inclined surface portion 22a are provided at the front side region Q2, whereby wear resistance performance in the front side region Q2 of the bottom surface 105s can be improved. Also, of the three land portion rows 11a to 11c, the land portion row 11a formed at the forefront in the sole front-rear direction Z is provided at a region including a front end in the sole front-rear direction Z of the bottom surface 105s.

As illustrated in FIG. 7, a plurality of land portions 14 and a plurality of land portions 15 which are defined by a plurality of grooves extending in the sole width direction W are arranged at the rear side region Q1 of the bottom surface 105s. The land portions 14 are arranged to the side of the toe T (see FIG. 1) from the land portions 15. The land portions 14 are shaped to include a width direction extending portion 14a extending in the sole width direction W to be substantially zigzag-shaped, a toe side protruding portion 14b extending to the side of the toe T (see FIG. 1) from a bent portion bending to be convex to the side of the toe T of the width direction extending portion 14a and a curved portion side protruding portion 14c extending to the side of the curved portion 3 from a bent portion bending to be convex to the side of the curved portion 3 (see FIG. 1) of the width direction extending portion 14a. The land portions 15 are shaped to include a width direction extending portion 15a, a toe side protruding portion 15b and a curved portion side protruding portion 15c. The width direction extending portions 14a and 15a are zig-zag shaped, thereby fully ensuring the edge component. Further, by forming the toe side protruding portions 14b and 15b as well as the curved portion side protruding portions 14c and 15c, the edge component is further increased, and the water film interposed between the bottom surface 105s and the road surface S can be efficiently cut on both sides in the sole front-rear direction Z, thereby achieving a high drainage performance.

In FIG. 7, a land portion width w3 of the width direction extending portion 15a of the land portions 15 is larger than a land portion width w2 of the width direction extending portion 14a of the land portions 14.

In this configuration, in the rear side region Q1, a percentage in an area of a groove portion which is concave to the road surface S in the planar view in a total area of the bottom surface 105s in the planar view, that is, a negative ratio is larger than that in the front side region Q2. Thus, in the rear side region Q1, more water can be taken in a recessed groove and can be discharged. Thus, the rear side region Q1 has a higher drainage performance than the front side region Q2.

On the other hand, the front side region Q2 has a higher wear resistance performance than the rear side region Q1. The reason is that the front side region Q2 has a smaller negative ratio than the rear side region Q1 to maintain a high rigidity.

Also, in FIG. 7, in the rear side region Q1, the negative ratio of the portion Q1-1 is larger than that of the portion Q1-2, so that more water can be taken in the grooves and can be discharged. In other words, the portion Q1-1 has a further higher drainage performance than the portion Q1-2.

Further, in the rear side region Q1, the land portions 15 are arranged in the portion Q1-2. Moreover, as described before, the land portion width w3 of the land portions 15 is larger than the land portion width w2 of the land portions 14. Thus, the portion Q1-2 has a larger land portion rigidity than the portion Q1-1. Further, the portion Q1-2 has a larger edge component in the width direction W than the portion Q1-1. Also, as described before, the negative ratio of the portion Q1-2 is smaller than that of the portion Q1-1.

Also, the portion Q1-2 has a larger edge component in the width direction W than the front side region Q2 and further, has a larger negative ratio.

Next, an attachment operation of attaching the sole 5 to the athletic prosthetic leg 1 as the prosthetic leg will be explained. The sole 5 illustrated in FIG. 1 is attached to the ground contact region 4s of the athletic prosthetic leg 1 by an adhesive. However, attachment means is not limited to the adhesive, and attachment may be executed using fasteners such as a belt. Further, while the sole 5 directly abuts the ground contact region 4s when it is attached in this embodiment, a cushion member (not illustrated) or an adhesive member may be interposed between the sole 5 and the ground contact region 4s.

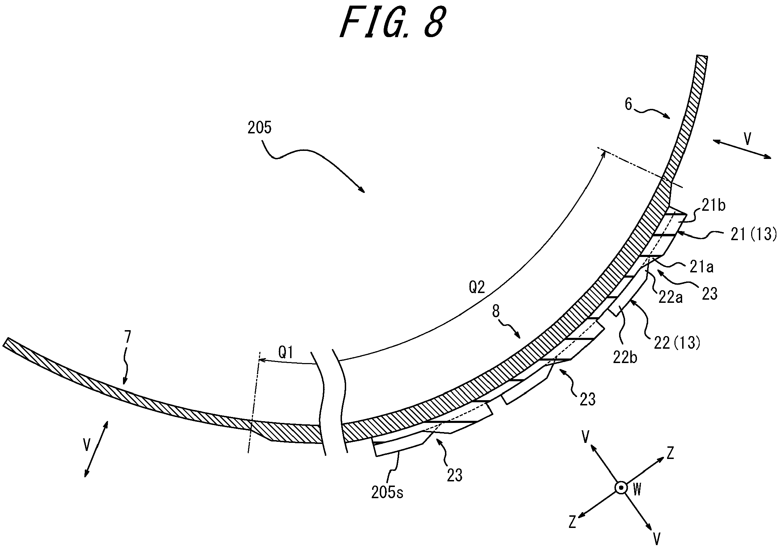

FIG. 8 is a drawing which illustrates an example of a sole 205 including a tab for sticking for attachment to the ground contact region 4s (FIG. 1) of the athletic prosthetic leg 1. In the sole 205 illustrated in FIG. 8, a front tab for sticking 6 and a rear tab for sticking 7 are provided at both ends in the sole front-rear direction Z of the sole 205. The front tab for sticking 6 and the rear tab for sticking 7 are portions extending in the sole front-rear direction Z from the sole body portion 8 corresponding to the rear side region Q1 and the front side region Q2 which are the ground contact region of a bottom surface 205s, and thicknesses in the sole thickness direction V of the front tab for sticking 6 and the rear tab for sticking 7 are thinner than a thickness in the sole thickness direction V of the ground contact region, that is, a thickness in the sole thickness direction V of the sole body portion 8. For example, assuming that the thickness of the sole body portion 8 is 2.25 to 3.0 mm, the thicknesses of the front tab for sticking 6 and the rear tab for sticking 7 may be 1.5 to 2.0 mm.

As illustrated in FIG. 8, the sole 205 is attached to the ground contact portion 4 (see FIG. 1) of the athletic prosthetic leg 1 in a deformed state along a curve of the ground contact region 4s (see FIG. 1) of the athletic prosthetic leg 1. More specifically, the tab for sticking 6 is folded at the side of the toe T to be fixed to an opposite surface to the ground contact region 4s of the ground contact portion 4. The rear tab for sticking 7 is fixed to the athletic prosthetic leg 1 at the heel side than the ground contact region 4s. Additionally, also in the sole 205 illustrated in FIG. 8, a portion corresponding to the ground contact region is adhered to the ground contact region 4s (see FIG. 1) by the adhesive etc.

Here, at least on the front side region Q2 of the bottom surface 205s of the sole 205, the plurality of land portions 13 each including the above-described first inclined surface portion 21a and the second inclined surface portion 22a are provided, whereby the continuous groove 23 extending in the sole width direction W is formed. With the continuous groove 23, out-of-plane deformation is easily executed along the ground contact portion 4 at a front side in the sole front-rear direction Z of the sole body portion 8 of the sole 205. Consequently, when the sole 205 is attached to the ground contact region 4s (see FIG. 1) of the athletic prosthetic leg 1, the front tab for sticking 6 is easily folded around the ground contact portion 4 while the front tab for sticking 6 is pulled toward the front side in the sole front-rear direction Z. In other words, by providing the continuous groove 23 extending in the sole width direction W at the front side region Q2 of the bottom surface 205s, compared with a configuration not including the continuous groove 23, the front tab for sticking 6 can be fixed around the ground contact portion 4 in a state of further close attachment. As a result, the sole body portion 8 can be closely attached to the ground contact region 4s (see FIG. 1), which inhibits occurrence of poor attachment of the sole 205.

The sole for a prosthetic leg according to the present disclosure is not limited to a specific configuration illustrated in the above-described embodiment, and various changes and modifications can be executed without departing from the description of the claims. For example, in any of the above-described embodiments, in the pattern of the bottom surface of the sole, fluorine is preferably applied to a groove wall and a groove bottom constituting a width direction groove which defines width direction land portions. Since the fluorine is applied to the groove wall and the groove bottom of the width direction groove, drainage performance in the bottom surface of the sole can be improved.

### EXAMPLES

While Examples of the present disclosure will be explained hereinafter, the present disclosure is not limited to this.

Prototypes are produced for each of soles of Examples and soles of comparative examples, and performance evaluation is executed. The soles of Examples are applied a function such as drainage performance specified in the present disclosure due to variation of an arrangement of the pattern or the grooves of the bottom surface of the sole. Of the soles of comparative examples, in a comparative example 1, a pattern of the sole is uniform at the bottom surface. Also, in a comparative example 2, a pattern is different from that of the present disclosure.

As for drainage performance and wear resistance performance, assuming that an index of Q1-1 of the comparative example 1 is 100, it is presented that the drainage performance and the wear resistance performance of the corresponding portion are excellent as the indexes increase.

The sole of comparative examples and the sole of Examples produced experimentally as described above are attached to the athletic prosthetic leg illustrated in FIG. 1 to evaluate anti-slip performance and wear resistance performance.

In the comparative example 1 and Example 4, drainage performance and wear resistance performance of each portion of each of the regions Q1, Q2 are evaluated from a result of calculation by simulation. Also, in the comparative example 2 and Examples 1 to 3, the drainage performance and the wear resistance performance of each portion of each of the regions Q1, Q2 are evaluated by the same method as in the comparative example 1 and Example 4.

### [Anti-slip property]

In a state that a water film of 1 mm is filled on a glass surface and a load of 980N is applied to an athletic prosthetic leg, the following test is executed. A spring scale is attached to a connection portion of the athletic prosthetic leg and a stump of a leg, and the athletic prosthetic leg is pulled to the side of the toe in the leg portion front-rear direction by the spring scale. At the time when the athletic prosthetic leg starts to slip, indexation of a value of the spring scale is executed.

Additionally, assuming that an index of the comparative example 1 is 100, it is presented that anti-slip property is excellent as the index increases.

### [Wear resistance performance]

A player with a healthy left leg wears an athletic prosthetic leg at a right side, and executes 200 km running on a public road, and thereafter, indexation of an appearance of the entire bottom surface is executed. Additionally, assuming that an index of the comparative example 1 is 100, it is presented that the sole has an excellent wear resistance performance as the index increases. In the comparative example 1 and Example 4, a player with a healthy left leg wore the athletic prosthetic leg at a right side, and executed 200 km running on a public road, and thereafter, indexation of an appearance of the entire bottom surface was executed. Also, in the comparative example 2 and Examples 1 to 3, indexation of the appearance of the entire bottom surface is executed by the same method as in the comparative example 1 and Example 4.

**[Table 1]**

| | | | | | | | | |
|---|---|---|---|---|---|---|---|---|
| <Example> | | | | | | | | |

| | | | Comparative example 1 | Comparative example 2 | Example 1 | Example 2 | Example 3 | Example 4 |
|---|---|---|---|---|---|---|---|---|
| Drainage performance | Rear side region Q1 | Portion Q1-1 | 100 | 100 | 110 | 110 | 110 | 120 |
| | | Portion Q1-2 | 100 | 100 | 110 | 110 | 110 | 110 |
| | Front side region Q2 | Portion Q2-1 | 100 | 110 | 80 | 90 | 90 | 90 |
| | | Portion Q2-2 | 100 | 110 | 50 | 90 | 90 | 90 |
| Wear resistance performance | Rear side region Q1 | Portion Q1-1 | 100 | 100 | 80 | 80 | 100 | 100 |
| | | Portion Q1-2 | 100 | 100 | 100 | 100 | 100 | 100 |
| | Front side region Q2 | Portion Q2-1 | 100 | 90 | 100 | 150 | 200 | 200 |
| | | Portion Q2-2 | 100 | 90 | 100 | 150 | 150 | 150 |
| Anti-slip performance | | | 100 | 103 | 110 | 110 | 110 | 120 |
| Wear resistance performance | | | 100 | 90 | 100 | 150 | 160 | 160 |

### REFERENCE SIGNS LIST

1 athletic prosthetic leg
2 leg portion
2a straight portion
2b, 2c curved portion
3 curved portion
4 ground contact portion
4s ground contact region
5, 205 sole
5s, 105s, 205s bottom surface
6 front tab for sticking
7 rear tab for sticking
8 sole body portion
11, 11a to 11g land portion row
12, 12a to 12f width direction groove
13 land portion
14, 15 land portion
14a, 15a width direction extending portion
14b, 15b toe side protruding portion
14c, 15c curved portion side protruding portion
21 first land portion
21a first inclined surface portion
21a1 first inclined surface
21b first body portion
22 second land portion
22a second inclined surface portion
22a1 second inclined surface
22b second body portion
23 continuous groove
D1 depth of a width direction groove
D2 depth of a continuous groove
Q1 rear side region
Q2 front side region
Q1-1, Q1-2, Q2-1, Q2-2 portion of a bottom surface
S road surface
T toe
V sole thickness direction
W width direction (sole width direction)
w0 width of a first inclined surface portion
w1 width of a second inclined surface portion
w2, w3 land portion width of a width direction extending portion of a land portion
XI, X2 arc
Y leg portion front-rear direction
Z sole front-rear direction
VL virtual line
θ inclination angle

## Claims

1. A sole for a prosthetic leg, the sole being configured to be attached to a ground contact region of the prosthetic leg, the sole comprising a bottom surface comprising:
a first inclined surface portion including a first inclined surface inclined such that an amount of recess in a sole thickness direction gradually increases from one side to the other side in a predetermined direction in a planar view; and
a second inclined surface portion including a second inclined surface inclined such that the amount of recess in the sole thickness direction gradually increases from the other side to the one side in the predetermined direction in the planar view,
wherein the first inclined surface portion and the second inclined surface portion are mutually connected in a connecting direction crossing the predetermined direction in the planar view, so that a continuous groove in which the first inclined surface and the second inclined surface constitute a part of a side wall and which extends in the connecting direction is defined.

2. The sole for the prosthetic leg according to claim 1, wherein, when a virtual line extending in parallel with a sole width direction at a center position in a sole front-rear direction is defined, the first inclined surface portion and the second inclined surface portion are provided at least on a front side region at a side of a toe with the virtual line as a border of the bottom surface.

3. The sole for the prosthetic leg according to claim 2, wherein a land portion row including a first land portion having the first inclined surface portion and a second land portion having the second inclined surface portion is provided at least on a region including a front end in the sole front-rear direction of the bottom surface.

4. The sole for the prosthetic leg according to any one of claims 1 to 3, wherein the predetermined direction is a sole front-rear direction.

5. The sole for the prosthetic leg according to any one of claims 1 to 4, wherein an inclination angle of the first inclined surface and the second inclined surface to the sole thickness direction is in a range of 40° to 80°.
